# EUROPÄISCHE PATENTSCHRIFT

(11) **EP 1 300 504 B1**
(45) Veröffentlichungstag und Bekanntmachung des Hinweises auf die Patenterteilung: **10.09.2008**
(21) Anmeldenummer: 01123929.0
(22) Anmeldetag: 06.10.2001
(51) Int. Cl.: D06H 3/08, G01N 21/898, G01N 33/36

(54) **Textilbahnsensor**
Textile web sensor
Capteur pour bande textile

(43) Veröffentlichungstag der Anmeldung: 09.04.2003
(73) Patentinhaber: Hergeth, Hubert A., 6300 Zug (CH)
(72) Erfinder: Hergeth, Hubert A., 6300 Zug (CH)

(56) Entgegenhaltungen:
- DE-C- 3 536 991
- US-A- 3 001 080
- US-A- 3 345 835
- US-A- 4 728 800

## Beschreibung

In der Textilindustrie ist es üblich, Warenbahnen mittels Zeilenkameras zu inspizieren.

In Webereien werden Stoffbahnen von einer Rolle abgewickelt und auf eine neue Rolle aufgewickelt oder getäfelt und dabei von einer oder mehreren Zeilenkameras inspiziert, und es werden Webfehler oder Fremdfasern festgestellt.

In der Vliesstoffindustrie werden Vliesstoffbahnen online mit einer oder mehreren Kameras inspiziert und auf Dünn- und Dickstellen, Löcher oder Flecken, kontrolliert. In der Spinnerei werden Flockenströme durch Rechteckkanäle geleitet, die auf der Längsseite durchsichtig sind, und der Flockenstrom wird durch einen oder mehrere Kameras auf Fremdfasern inspiziert.

An Baumwollkarden ist eine Vorrichtung bekannt, die aus einem länglichem Aluminiumgehäuse besteht, das an einer Längsseite eine Glasabdeckung aufweist und in deren Inneren sowohl eine kleine Kamera als auch einige Leuchtdioden auf einer Verfahrvorrichtung in dem Gehäuse hin und her bewegt werden.

Mit dieser Vorrichtung werden die Fasern auf dem Doffer der Baumwollkarde auf das Auftreten von Nissen statistisch untersucht.

Eine ganz ähnliche Vorrichtung wird in der Weberei an der Webmaschine eingesetzt, um dort ziemlich direkt nach dem Einbindevorgang Webfehler zu detektieren. Bei denen in einem Gehäuse bewegten Kameras können immer nur Teilbereiche des zu betrachtenden Objekts mit der bewegten Kamera pro Zeiteinheit erfasst werden.

Bei den stationären Kamerasystemen ist der große Abstand zwischen Kamera und Textiloberfläche von Nachteil.

Das US-4,728,800 zeigt ein Sensorgehäuse, das abgedichtet ist, jedoch keine Beleuchtung und Linsen beinhaltet.

Die DE-3636991 zeigt eine punktuelle Inspektion an einer Rundstrickmaschine.

Die US-3,001,080 zeigt eine Bahninspektion, bei der Schlitze im Gehäuse zur Größenbestimmung der Fehler dienen.

Die Objektive und die Beleuchtung verschmutzen durch die in dieser Industrie üblichen Staub und Fasern; der große Abstand führt zu Problemen beim Einbau solcher Anlagen, und die Justierung der Kameras ist schwierig.

Aufgabe der Erfindung ist es, ein Sensorsystem zu verbessern, bei dem sich eine Reihe von lichtempfindlichen Elementen in etwa über die ganze Breite der zu inspizierenden Faserbahn erstreckt und selbige abtastet.

Das Sensorsystem besteht aus einem Gehäuse (1), an dessen Oberfläche eine Glasplatte (2) eingebettet ist.

Die Glasplatte ist bündig oder steht etwas über das Gehäuse hinaus, damit die Faserbahn (9) die Oberfläche der Glasscheibe durch Berührung sauber halten kann.

Die Glasscheibe kann auch leicht gewölbt sein, um diesen Effekt zu erhöhen.

Die Glasplatte und das Gehäuse bilden eine staubdichte Einheit.

In dem Gehäuse befindet sich eine Anordnung von Stablinsen oder ein stabförmiges Objektiv (5) das die durch die Glasscheibe empfangenen Lichtstrahlen auf die Sensorleiste (6) leitet.

Linse und Sensorleiste sind auf jeweils einer Höhenverstellvorrichtung (4), (7) angebracht. Zwischen Linse und gegenüberliegender Gehäusewand ist eine Dichtung (8), die ein Einfallen von Streulicht auf die Sensoren verhindert.

Eine Beleuchtung (3) erstreckt sich etwa über die ganze Länge der Sensorleiste. Die Beleuchtung sind z. B. mindestens eine Leuchtstoffröhre oder Leuchtdioden.

Das Gehäuse (1) besteht vorteilhaft aus einem Aluminiumstrangusprofil. In dem Gehäuse kann auch eine Datenvorverarbeitung der einzelnen Werte der Sensorpixel erfolgen.

Bei Bahnen, die besser im Durchlicht beurteilt werden können, kann die Sensorleiste ohne Beleuchtung arbeiten und die Beleuchtung von einer gegenüberliegenden Lichtquelle erfolgen, oder das Licht kann über einen auf der anderen Seite der Faserbahn angebrachten Spiegel in das Gehäuse zurückscheinen.

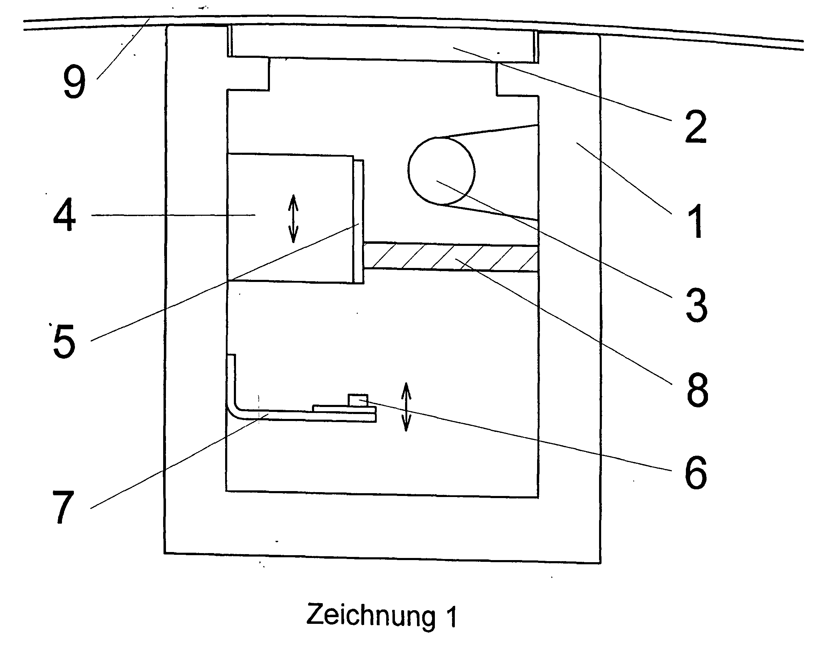

## Patentansprüche

1. Vorrichtung zum Beobachten von flexiblem Material (9), die ein gegen Staub abgedichtetes Gehäuse (1), eine Glasscheibe (2), die direkt oder über eine Zwischenscheibe Kontakt mit dem beobachteten Material hat, **dadurch gekennzeichnet, daß** sich in der Vorrichtung ein Linsensystem (5), eine Beleuchtungseinrichtung (3), die sich in etwa über die Länge des Gehäuses (1) erstreckt und eine Reihe Sensorelemente (6), die in etwa so lang ist wie die Glasfläche (2) der Vorrichtung, befinden.

2. Vorrichtung nach Anspruch 1) **dadurch gekennzeichnet, daß** die Glasfläche (2) gewölbt ist.

## Claims

1. Device for inspecting a flexible width of material comprising a housing that is sealed against dust and a glass window that has direct contact with the inspected material **characterized by** the fact that inside the device is an illumination unit that stretches about the length of the housing and an array of sensor elements that is approximately the length of the glass window.

2. Device according to claim 1 **characterized by** the window being cambered.

## Revendications

1. apparatus pour examiner du matériel flexible qui dispose d'un boîtier étanche contre la poussière et d'une vitre ayant contact directe ou par une vitre intermédiaire avec le matériel à examiner **caractérisé par le fait que** endéans du boîtier se trouvent un système de lentilles, une installation d'illumination et un alignement de capteurs qui sont à peu près la même longueur que la vitre.

2. Apparatus selon titre 1 carcatérisé par le fait que la vitre présente une voûte.
